# EUROPEAN PATENT APPLICATION

(11) **EP 4 336 519 A1**
(43) Date of publication of application: **13.03.2024**
(21) Application number: 23195886.9
(22) Date of filing: 07.09.2023
(51) Int. Cl.: G16H 80/00

(54) **PROVIDING REMOTE PATIENT ASSISTANCE WHEN NETWORK CONNECTIVITY IS UNAVAILABLE**

(30) Priority: 07.09.2022 IN 202241051078
(71) Applicant: Harman International Industries, Incorporated, Stamford, CT 06901 (US)
(72) Inventor: ANAND, Ankush, Stamford, 06901 (US); BHASIN, Manish Kumar, Stamford, 06901 (US); CHAUDHARI, Rahul R., Stamford, 06901 (US); BEHERA, Arabinda Ranjan, Stamford, 06901 (US); GUPTA, Anuj, Stamford, 06901 (US)
(74) Representative: Rummler, Felix

(57) **Abstract**

Techniques providing remote patient assistance when network connectivity is unavailable include receiving, from a provider-side side remote patient monitoring (RPM) platform, a healthcare plan assigned to a patient; and while disconnected from the provider-side RPM platform: monitoring the patient; in response to the monitoring, determining whether to provide the medical information to the patient; and presenting the medical information to the patient, wherein the medical information is based on the healthcare plan and the monitoring.

## Description

### BACKGROUND

### Field of the Various Embodiments

Embodiments of the present invention relate generally to providing healthcare services and, more specifically, to providing remote patient assistance when network connectivity is unavailable.

### Description of the Related Art

Remote health monitoring services provided via mobile applications have played a significant role in transformation of the healthcare sector. Using mobile applications, which are frequently installed on smartphones, healthcare providers (e.g., doctors and nurses) can easily connect with their patients after the patients are discharged from the hospital or leave the office and continue to provide healthcare remotely. The remote health monitoring services can involve videocalls between patients and healthcare providers for consultations and diagnoses, as well as monitoring of patient vital signs (e.g., blood pressure, pulse rate, and/or the like) via healthcare monitoring devices (e.g., wearable healthcare monitoring devices) that report patient information to the mobile applications, which provide the patient information to the remote healthcare providers via wireless or wired communication networks.

Remote health monitoring services provided via devices with wide-area network (WAN) connections are well suited for the delivery of relevant medical information when a patient has an emergency or exigency, as the connected devices can easily connect to a healthcare professional who is familiar with the condition of the patient. However, a drawback of using WAN-connected devices to provide the health monitoring services is that patients with no or limited WAN connectivity (e.g., people engaged in jobs like mining, petroleum extraction, seafaring, warehousing, and underground storage) have difficulties getting relevant medical information for emergency conditions or exigencies, when the patient is most in need of the relevant medical information. These difficulties can include an inability to receive appropriate relevant medical information because the patient is unable to contact a healthcare professional (e.g., a doctor, nurse, or nurse practitioner) familiar with the patient's current medical information and medical history. For these reasons, remote health monitoring services are often unsuitable for patients with no or limited WAN connectivity.

As the foregoing illustrates, what is needed in the art are systems to provide remote patient assistance when network connectivity is unavailable.

### SUMMARY

Various embodiments of the present disclosure set forth a computer-implemented method for providing relevant medical information to a patient. The method includes receiving, from a provider-side side remote patient monitoring (RPM) platform, a healthcare plan assigned to a patient; and while disconnected from the provider-side RPM platform: monitoring the patient; in response to the monitoring, determining whether to provide the medical information to the patient; and presenting the medical information to the patient, wherein the medical information is based on the healthcare plan and the monitoring.

Further embodiments provide, among other things, one or more non-transitory computer-readable media and systems configured to implement the method set forth above.

At least one technical advantage of the disclosed techniques relative to the prior art is that, with the disclosed techniques, a patient without connectivity to a provider-side RPM platform can be monitored and receive remote patient assistance based on the monitoring and a healthcare plan personalized for the patient by a healthcare professional. This enables the patient to receive relevant and timely medical information even when the patient does not have access to a healthcare professional or other remote resources. Another technical advantage of the disclosed techniques relative to the prior art is that, with the disclosed techniques, a healthcare professional can update a healthcare plan for a patient without regard to whether the patient has connectivity (e.g., via a WAN) to the provider-side RPM platform to receive the updated healthcare plan. This enables the healthcare professional to update the healthcare plan at a time chosen by the healthcare professional while ensuring that the patient will receive the update as soon as the patient has connectivity to the provider-side RPM platform. These technical advantages provide one or more technological improvements over prior art approaches.

### BRIEF DESCRIPTION OF THE DRAWINGS

So that the manner in which the above recited features of the various embodiments can be understood in detail, a more particular description of the inventive concepts, briefly summarized above, may be had by reference to various embodiments, some of which are illustrated in the appended drawings. It is to be noted, however, that the appended drawings illustrate only typical embodiments of the inventive concepts and are therefore not to be considered limiting of scope in any way, and that there are other equally effective embodiments.
Figure 1 is a schematic diagram of a remote patient monitoring (RPM) system configured to implement one or more aspects of the present disclosure;
Figure 2 illustrates an example of a graphical user interface (GUI) for configuring a healthcare plan, according to various embodiments of the present disclosure;
Figure 3 illustrates an example of a GUI for configuring a healthcare plan, according to various embodiments of the present disclosure;
Figures 4A and 4B illustrate a flow diagram of method steps for remote patient assistance, according to various embodiments of the present disclosure; and
Figure 5 illustrates a flow diagram of method steps managing a healthcare plan for providing remote patient assistance, according to various embodiments of the present disclosure.

### DETAILED DESCRIPTION

In the following description, numerous specific details are set forth to provide a more thorough understanding of the various embodiments. However, it will be apparent to one skilled in the art that the inventive concepts may be practiced without one or more of these specific details.

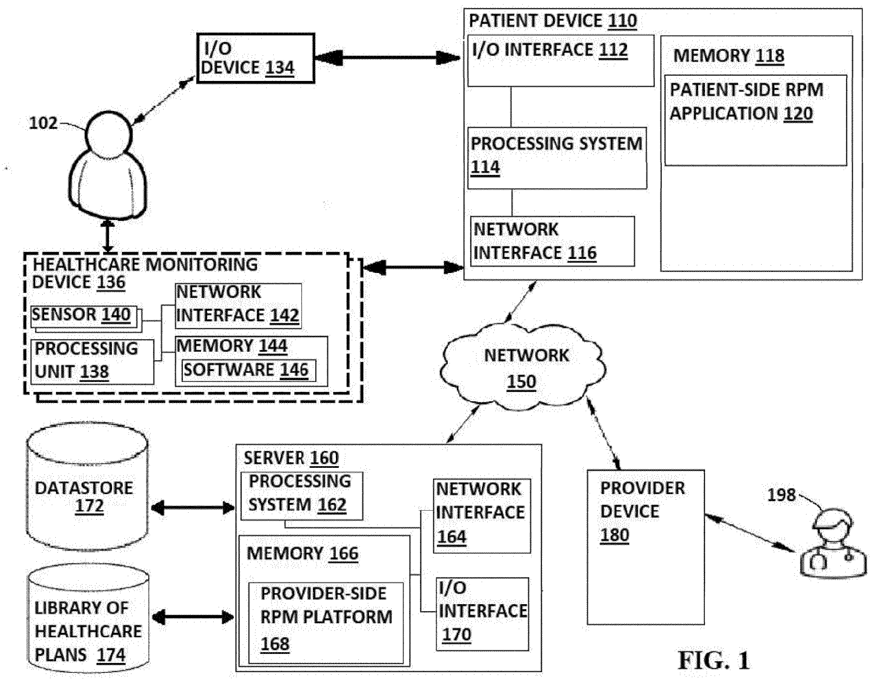

Figure 1 is a schematic diagram of a remote patient monitoring (RPM) system 100 configured to implement one or more aspects of the present disclosure. RPM system 100 provides relevant medical information (e.g., patient-specific healthcare information) to a patient 102 according to a healthcare plan selected and/or configured by a healthcare provider 198 (e.g., a doctor, nurse practitioner, or nurse). The RPM system 100 includes, without limitation, a patient device 110, a server 160, a datastore 172, a library of healthcare plans 174, and a provider device 180. The devices of the RPM system 100 communicate with each other or via a network 150 (e.g., the Internet). In some examples, the RPM system 100 further includes one or more healthcare monitoring devices 136.

The patient device 110 is a computer-based system that interacts with the patient 102 via various devices, including, without limitation, the one or more healthcare monitoring devices 136. The patient device 110 includes, without limitation, an input/output (I/O) interface 112, a processing system 114, a network interface 116, and a memory 118. In some embodiments, the patient device 110 is a tablet computer, a laptop computer, a smartphone, a smartwatch, and/or the like. The patient device 110 can include any technically feasible type of computer system.

The I/O interface 112 facilitates connectivity between the patient device 110 and I/O devices. The I/O interface can include one or more of a high-definition multimedia interface (HDMI), a universal serial bus (USB) interface, an infrared (IR) interface, a radio frequency (RF) interface, a serial interface, a parallel interface, and/or the like. Examples of I/O devices the patient device 110 can communicate with via the I/O interface 112 include, without limitation, a camera (which can include a microphone), a display device , a pointer device (e.g., a mouse), speakers, and a touch screen.

Processing system 114 generally comprises a programmable processor that executes program instructions to manipulate input data and generate output data. Processing system 114 can be implemented as a central processing unit (CPU), a digital signal processing unit (DSP), a microprocessor, an application-specific integrated circuit (ASIC), a neural processing unit (NPU), a graphics processing unit (GPU), a field-programmable gate array (FPGA), and/or any other type of processing unit, or a combination of different processing units. The processing system 114 of the patient device 110 executes a patient-side remote patient monitoring application 120 that is stored in the memory 118.

Network interface 116 enables the patient-side RPM application 120 to communicate with the network 150 and via the network with a provider-side RPM platform 168 executing on a server 160. Network interface 116 also enables the patient-side RPM application 120 to communicate with one or more healthcare monitoring devices 136 (e.g., a FitBit^{™}). Network interface 116 can implement any technically feasible wired or wireless communications protocol to facilitate the communications with the network 150 and/or the healthcare monitoring devices 136. For example, network interface 116 can include one or more of an optical network interface, a cable-network interface, an ethernet interface, a Wi-Fi transceiver, a Bluetooth^{™} transceiver, and/or the like.

Memory 118 includes a memory module or a collection of memory modules. Memory 118 can include a variety of computer-readable media selected for their size, relative performance, or other capabilities: volatile and/or non-volatile media, removable and/or non-removable media, and/or the like. Memory 118 can include cache, random access memory (RAM), storage, and/or the like. Memory 118 can include one or more discrete memory modules, such as dynamic RAM (DRAM) dual inline memory modules (DIMMs). In various embodiments, the non-volatile memory includes flash memory, optical drives, magnetic drives, flash drives, and/or other non-volatile storage. In some embodiments, separate data stores (not shown), can supplement memory 118.

Memory 118 generally stores application programs including patient-side RPM application 120, and data for processing by processing system 114. In some embodiments, memory 118 includes various software programs and modules (e.g., an operating system, one or more applications, and/or the like) that can be executed processing system 114 and application data associated with the software program. As shown, memory 118 includes at least the patient-side RPM application 120 that is executed by processing system 114 to implement the overall functionality of patient device 110 related to providing relevant medical information as discussed in greater detail herein.

Patient-side RPM application 120 configures the healthcare monitoring devices 136 and receives healthcare data captured by the healthcare monitoring devices 136. In some examples, the RPM application 120 uses an API provided by the software 146 of the healthcare monitoring device 136 to request the healthcare data from the healthcare monitoring device 136 at periodic intervals, in response to various alerts, in response to notifications received from the healthcare monitoring device 136, and/or in response to notifications received from another healthcare monitoring device 136. As illustrated, the patient-side RPM application 120 communicates with the healthcare monitoring devices 136 using the network interface 116. For example, the patient-side RPM application 120 can communicate with the healthcare monitoring devices 136 via any feasible wired (e.g., USB) or wireless (e.g., Wi-Fi or Bluetooth^{™}) networking protocol. The patient-side RPM application 120 stores the received healthcare data in the memory 118 or alternatively in separate storage (not shown). When the network 150 provides connectivity to the provider-side RPM platform 168, the patient-side RPM application 120 also sends the healthcare data to the provider-side RPM platform 168. In cases in which the network 150 does not provide connectivity to the provider-side RPM platform 168, when the patient-side RPM application 120 receives healthcare data (e.g., from the healthcare monitoring devices 136 or from the patient 102), the patient-side RPM application 120 stores the healthcare data so that the healthcare data can be sent to the provider-side RPM platform 168 when the network 150 provides connectivity to the provider-side RPM platform 168.

The patient-side RPM application 120 communicates with the provider-side RPM platform 168 via the network 150 using network interface 116, when the network 150 provides connectivity. The patient-side RPM application 120 receives a healthcare plan from the provider-side RPM platform 168. In some examples, the patient-side RPM application 120 sends a confirmation of the receipt of the healthcare plan to the provider-side RPM platform 168 after receiving a healthcare plan from the provider-side RPM platform 168. If the healthcare plan is updated (e.g., by a healthcare provider editing the healthcare plan via the provider-side RPM platform on the server 160) while the network 150 does not provide connectivity to the provider-side RPM platform 168, the patient-side RPM application 120 updates the healthcare plan stored at the patient device 110 when the network 150 next provides connectivity to the provider-side RPM platform 168. The patient-side RPM application 120 sends healthcare data (e.g., healthcare data received from the healthcare monitoring devices 136 and stored in the memory 118) to the provider-side RPM platform 168, when the network 150 provides connectivity to the provider-side RPM platform 168.

The patient-side RPM application 120 communicates with one or more I/O devices 134 via the I/O interface 112 and/or the network interface 116. The I/O device(s) 134 can include one or more devices capable of obtaining input for the patient-side RPM application 120, such as a touchpad, a touch-sensitive screen, buttons, knobs, dials, sliders, joysticks, microphones, cameras, and so forth, as well as devices capable of providing output to the patient 102, such as a display device, audio speaker, indicator lights, and the like. Examples of display(s) include, without limitation, LCD displays, LED displays, touch-sensitive screens, transparent displays, projection systems (e.g., a heads-up display projection system), optical combiners, and/or the like. Additionally, I/O device(s) 134 can include devices capable of both receiving input and providing output, such as a touch-sensitive screen, a universal serial bus (USB) port, and/or the like. In some examples, the patient device 110 includes one or more of the I/O device(s) 134 integrated within the patient device 110 and able to communicate with the processing system 114 via an internal bus of the patient device 110.

The patient-side RPM application 120 communicates with the I/O device(s) 134 to present information to and obtain information from the patient 102. In some examples, the information includes content related to the healthcare plan (e.g., after-visit summaries, educational material, questionnaires, and/or the like). The patient-side RPM application 120 presents healthcare reminders and/or healthcare alerts as needed based on the healthcare plan and patient monitoring data. The patient-side RPM application 120 provides the reminders and/or healthcare alerts whether or not the network 150 provides connectivity to the provider-side RPM platform 168. In some examples, the patient-side RPM application 120 receives images of the patient 102 (e.g., using a camera) and audio from the patient 102 (e.g., using a microphone) to facilitate video calls with a healthcare provider, and/or the like. The patient-side RPM application 120 receives input (e.g., healthcare information, such as vital signs that are not directly collected from a healthcare monitoring device 136 and answers to questions) from the patient 102, such as when used by the patient 102 to navigate and interact with a user interface presented by a user interface of the patient-side RPM application 120.

The patient-side RPM application 120 provides relevant medical information to the patient 102 for responding to emergencies, exigencies, and emergent situations according to the healthcare plan assigned to the patient 102 and based on healthcare data from healthcare monitoring devices 136. The patient-side RPM application is able to provide the relevant medical information to the patient even when the network 150 does not provide connectivity to the provider-side RPM platform 168.

The server 160 is a computer-based system that supports the provider-side functionality of the RPM system 100. The server 160 includes, without limitation, a processing system 162, a memory 166, a network interface 164, and an I/O interface 170. And although server 160 is shown as a single server, it is understood that the functionality provided by server 160 can be provided by two or more servers, one or more virtual machines as part of a server, as part of a cloud-based infrastructure, and/or the like.

Processing system 162 generally comprises a programmable processor that executes program instructions to manipulate input data. Processing system 162 can be implemented as a central processing unit (CPU), a digital signal processing unit (DSP), a microprocessor, an application-specific integrated circuit (ASIC), a neural processing unit (NPU), a graphics processing unit (GPU), a field-programmable gate array (FPGA), and/or any other type of processing unit, or a combination of different processing units.

Network interface 164 enables the provider-side RPM platform 168 to communicate with the patient-side RPM application 120 and one or more healthcare providers 198 using network 150. In some embodiments, the network interface 164 also enables the provider-side RPM platform 168 to communicate with the healthcare monitoring devices 136 via network 150. Network interface 164 can implement any technically feasible wired or wireless communications protocol to facilitate the communications with the network 150 and/or the healthcare monitoring devices 136. For example, network interface 164 can include one or more of an optical network interface, a cable-network interface, an ethernet interface, a Wi-Fi transceiver, a Bluetooth^{™} transceiver, and/or the like.

Memory 166 includes a memory module or a collection of memory modules. Memory 166 can include a variety of computer-readable media selected for their size, relative performance, or other capabilities: volatile and/or non-volatile media, removable and/or non-removable media, and/or the like. Memory 166 can include cache, random access memory (RAM), storage, and/or the like. Memory 166 can include one or more discrete memory modules, such as dynamic RAM (DRAM) dual inline memory modules (DIMMs). In various embodiments, the non-volatile memory includes flash memory, optical drives, magnetic drives, flash drives, and/or other non-volatile storage.

Memory 166 generally stores application programs including provider-side RPM platform 168, and data for processing by processing system 162. In some embodiments, memory 166 includes various software programs and modules (e.g., an operating system, one or more applications, and/or the like) that can be executed processing system 114 and application data associated with the software program. As shown, memory 166 includes at least the provider-side RPM platform 168.

In some embodiments, separate data stores such as datastore 172 supplement memory 166. In some examples, datastore 172 includes storage for various libraries and/or other data sources to support operation of RPM system 100. In some examples, datastore 172 is implemented on one or more storage devices (e.g., disk drives, databases, and/or the like) that are coupled to the server 160. Additionally and/or alternatively datastore 172 can be coupled to server 160 through network 150 and can include network attached storage, cloud-based storage, and/or the like.

I/O interface 170 enables the provider-side RPM platform 168 to communicate with various peripheral devices to communicate with the healthcare provider 198. Examples of devices the provider-side RPM platform 168 can communicate with via the I/O interface 170 include, without limitation, a camera (which can include a microphone), a display, a media device (which can include speakers), a keyboard, and/or a touchscreen.

The provider-side RPM platform 168 provides one or more user interfaces that are usable by a healthcare provider 198 to configure a healthcare plan assigned to the patient 102. For example, the provider-side RPM platform 168 can retrieve disease-specific or diagnosis-specific healthcare plans from a library of healthcare plans 174 and present the retrieved healthcare plans to the healthcare provider 198 for the healthcare provider 198 to use in configuring and/or editing the healthcare plan assigned to the patient 102. The provider-side RPM platform 168 also retrieves healthcare data for the patient from datastore 172 containing patient-assigned healthcare plans and patient-specific healthcare data. At the direction of the healthcare provider 198, when the healthcare provider 198 has completed creating or updating the healthcare plan, the provider-side RPM platform 168 transmits the healthcare plan to the patient-side RPM application 120 on patient device 110. In cases in which the patient device 110 is not in communication with the provider-side RPM platform 168 (e.g., the network does not provide connectivity to the provider-side RPM platform 168), the provider-side RPM platform 168 stores the healthcare plan (e.g., in the datastore 172) and transmits the healthcare plan to the patient-side RPM application 120 when the patient device 110 next connects with the provider-side RPM platform 168 via the network 150.

The provider-side RPM platform 168 further receives healthcare data for the patient 102 from the patient-side RPM application 120. The provider-side RPM platform 168 stores the received healthcare data for the patient 102 in the datastore 172. The provider-side RPM platform 168 analyzes the received healthcare data for the patient 102 to determine whether to send an alert to the healthcare provider 198 regarding an update and/or a change in health status of patient 102.

The datastore 172 stores healthcare data and one or more healthcare plans of the patient 102. The datastore 172 can include non-volatile memory, such as optical drives, magnetic drives, flash drives, and/or other non-volatile storage.

The library of healthcare plans 174 stores one or more healthcare plans that are intended to be used and can be customized for any patient 102. The library of healthcare plans 174 can include non-volatile memory, such as optical drives, magnetic drives, flash drives, or other storage. The healthcare provider 198 can refer to healthcare plans in the library of healthcare plans when creating or updating a healthcare plan assigned to the patient 102.

Each healthcare monitoring device 136 includes, without limitation, a processing unit 138, a memory 144, a network interface 142, and one or more sensors 140. The healthcare monitoring devices 136 each receive a configuration from the patient-side RPM application 120 and/or from the provider-side RPM platform 168, sense the patient 102 to collect healthcare data based on the configuration, store the healthcare data, and send the healthcare data to the patient-side RPM application 120 and/or the provider-side RPM platform 168.

Processing unit 138 can include a central processing unit (CPU), a digital signal processing unit (DSP), a microprocessor, an application-specific integrated circuit (ASIC), a neural processing unit (NPU), a graphics processing unit (GPU), a field-programmable gate array (FPGA), and/or any other type of processor, or a combination of different processors. Processing unit 138 generally comprises a programmable processor that executes program instructions to manipulate input data. In some embodiments, processing unit 138 can include any number of processing cores, memories, and other modules for facilitating program execution.

Memory 144 includes a memory module, or collection of memory modules. Memory 144 can include a variety of computer-readable media selected for their size, relative performance, or other capabilities: volatile and/or non-volatile media, removable and/or non-removable media, and/or the like. Memory 144 can include cache, random access memory (RAM), storage, and/or the like. Memory 144 can include one or more discrete memory modules, such as dynamic RAM (DRAM) dual inline memory modules (DIMMs). In various embodiments, non-volatile memory, such as optical drives, magnetic drives, flash drives, and/or other types of non-volatile storage. In some embodiments, separate data stores can supplement memory 144.

Memory 144 generally stores application programs and data for processing by processing unit 138. In various embodiments, memory 144 stores software 146 for performing various functions or techniques described herein. The software 146 provides application program interfaces (APIs) for the healthcare monitoring device 136. The APIs are usable by the patient-side RPM application 120 and/or the provider-side RPM platform 168 to configure, access, and use the functionality provided by the healthcare monitoring device 136.

The processing unit 138 of each healthcare monitoring device 136 controls the one or more sensors 140 to collect healthcare data regarding the patient 102 based on the configuration received from the patient-side RPM application 120 or the provider-side RPM platform 168. The processing unit 138 of each healthcare monitoring device 136 also stores the healthcare data collected by the one or more sensors 140 in the memory 144. The processing unit 138 of each healthcare monitoring device 136 also sends, via the network interface 142, the stored healthcare data to the patient-side RPM application 120 or the provider-side RPM platform 168.

The one or more sensors 140 collect healthcare data of the patient 102. For example, the one or more sensors 140 sense one or more of the pulse rate, blood pressure, temperature, electroencephalograms (EEGs), electrocardiograms (EKGs), rapid eye movement (REM), physical activity (e.g., exercising, walking, climbing stairs), oxygenation level of the patient 102, and/or the like. The healthcare data measured by the one or more sensors is read by the processing unit 138, which stores the sensed healthcare data in the memory 144 and/or takes other actions (e.g., change a configuration of a sensor 140) in response to the sensed healthcare data.

The provider device 180 enables the healthcare provider 198 to interact with the provider-side RPM platform 168 and/or the patient-side RPM application 120. The provider device 180 interacts with the provider-side RPM platform 168 to enable the healthcare provider 198 to create, review, update, or delete a healthcare plan assigned to the patient 102. The provider device 180 also interacts with the provider-side RPM platform 168 to enable the healthcare provider 198 to review healthcare data (e.g., records of vital signs) of the patient 102 and/or to have a video call with the patient 102. The provider device 180 interacts with the patient-side RPM application 120 to review healthcare data (e.g., current vital signs) of the patient 102 and/or to have a video call with the patient 102. The provider device 180 can be a smartphone, tablet, laptop, or any other type of computing device capable of communicating via network 150.

The patient-side RPM application 120 receives a healthcare plan from the provider-side RPM platform 168 via the network 150, stores the healthcare plan in the memory 118 of the patient device 110, and parses the healthcare plan. The patient-side RPM application 120 parses the healthcare plan to identify and process offerings of content to the patient 102, monitoring configurations, event configurations, state configurations, reminder configurations, and/or alert configurations.

In some embodiments, the patient-side RPM application 120 configures the optional healthcare monitoring devices 136 according to a healthcare plan received by the patient-side remote patient monitoring application 120. Configuring a healthcare monitoring device 136 includes, without limitation, sending instructions to the healthcare monitoring device 136 to collect healthcare data regarding the patient 102 at particular times, at regular intervals, in response to detected events, or in response to detected states. In some examples, the patient-side RPM application 120 uses the one or more APIs of the healthcare monitoring device 136 to configure the healthcare monitoring device 136. Depending upon the capabilities of the healthcare monitoring device 136, the patient-side RPM application 120 can configure the healthcare monitoring device 136 to collect data from different sensors 140 either operating together or independently from each other. In some embodiments, the patient-side RPM application 120 configures a healthcare monitoring device 136 to collect a first type of healthcare data with a first sensor 140 at a first set of times and to collect a second type of healthcare data with a second sensor 140 at a second set of times. The intervals between the first set of times can be different than the intervals between the second set of times, resulting in the first sensor 140 collecting healthcare data a number of times during a period and the second sensor 140 collecting healthcare data a different number of times during the same period.

The patient-side RPM application 120 receives healthcare data from healthcare monitoring devices 136. In some examples, the patient-side RPM application 120 receives an indication of an emergent health condition of the patient 102 from one or more of the healthcare monitoring devices 136. The patient-side RPM application 120 logs the healthcare data in the memory 118 in the patient device 110. The patient-side RPM application 120 can additionally or alternatively forward the healthcare data to the provider-side RPM platform 168.

A healthcare plan can be assigned to the patient 102 by a healthcare provider 198 and can be customized for the patient 102 by the healthcare provider 198. A healthcare plan includes, without limitation, offerings of content to the patient 102, questionnaires, monitoring configurations, event configurations, state configurations, reminder configurations, and/or alert configurations.

The offerings of content in a healthcare plan includes information associated with the healthcare plan that a healthcare provider (e.g., healthcare provider 198) would like to make available to a patient (e.g., patient 102). For example, the content could include one or more of an after-visit summary, a set of instructions, and/or third-party content, such as web pages, audio, video, and/or the like. The content can either be included in the healthcare plan or identified using links, such as a universal resource locator (URL). When the healthcare plan includes content to be offered to the patient, the patient-side RPM application 120 downloads the content to the memory 118 of the patient device 110. The patient-side RPM application 120 offers the content to the patient when the download is complete or at a later time. For example, the patient-side RPM application 120 can display a user interface using a display device with a list of available (e.g., downloaded) content. The patient can use an I/O device (e.g., a remote control, a microphone, a mouse, or a touchscreen) to select the content to consume. The patient-side RPM application 120 then presents the content to the patient via an output device (e.g., a video screen and/or speakers). When the content is identified by a link or URL, the patient-side RPM application 120 uses the link or URL to download the content in order to present the downloaded content to the patient. In some embodiments, if the patient 102 does not consume the content (e.g., watch an offered video or listen to an audio file) when the content is offered, the patient-side RPM application 120 can configure a reminder for the patient 102 to consume the content at a later time.

A questionnaire includes, without limitation, one or more questions for the patient to answer and suggested answers (if any) for the questions. Both the questions and the suggested answers are selected by a healthcare provider when the healthcare provider configures the healthcare plan assigned to the patient. The questions in a questionnaire are designed to solicit input from the patient that can assist the patient-side RPM application 120 in identifying relevant medical information to the patient in response to a possible emergency, exigency, and/or the like being experienced by the patient. For examples, the questions can solicit information that is difficult to obtain from healthcare monitoring devices, such as information about a type, level, and/or location of pain, whether the patient is experiencing dizziness, and the like. The healthcare provider can select from questions and answers stored in a library and/or suggested by the provider-side RPM platform 168 based on the history and other healthcare data of the patient. The healthcare provider can also generate questions and answers (e.g., for multiple-choice questions) based on the healthcare data of the patient and the professional judgment of the healthcare provider. The patient-side RPM application 120 presents a questionnaire to the patient based on monitoring of vital signs of the patient or in response to a request or other input from the patient. In some cases, the questionnaire is configured so that the patient providing or selecting a particular answer to a question causes the patient-side RPM application 120 to ask a particular question or skip a particular question (e.g., if a patient reports no pain, then additional questions about pain in particular locations are skipped). In some cases, answers provided by the patient are used to select other content and/or questionnaires to be presented to the patient.

An event configuration includes, without limitation, a definition for detecting that a healthcare event has occurred and a set of healthcare data to be collected and/or reported in response to detecting that the event has occurred. Examples of events that can be defined in event configurations include, without limitation, a vital sign of the patient 102 going above or below a threshold, the patient 102 falling, the patient 102 beginning or ceasing activities (e.g., exercise, climbing stairs, eating, sleeping, walking, running), and/or the like.

A state configuration includes, without limitation, a definition for detecting the patient is in a particular physiological state and a set of vital signs to be reported while the patient is in the physiological state. Examples of physiological states that can be defined in state configurations include, without limitation, being asleep, being in rapid eye movement (REM) during sleep, exercising, walking, climbing stairs, being in labor, and/or the like.

A reminder configuration causes the patient-side RPM application 120 to present a reminder to the patient 102. The patient-side RPM application 120 causes the reminder to be presented via the I/O device(s) 134 and/or the like. The patient-side RPM application 120 can cause the reminder to be presented beside other content on a display device or in a pop-up window over the other content on the display device. The patient-side RPM application 120 can interrupt other content on the display device with the reminder and/or pause the other content while presenting the reminder. In some examples, a reminder also includes an audio component, such as a tone, a distinctive sound, a verbal component, and/or the like. A reminder includes, without limitation, a message regarding taking a medication, a message regarding an appointment with the healthcare provider 198, a message regarding an activity (e.g., sleeping, eating, or exercising) of the patient 102, a message regarding an offering of content to the patient 102, and/or the like.

An alert configuration causes the patient-side RPM application 120 to present relevant medical information to the patient 102 and/or send an alert to the healthcare provider 198. The alert configuration has one or more criteria for triggering the alert and a severity of the alert (e.g., critical, high, or low). Optionally, the alert configuration can include a message to include when sending the alert to the healthcare provider 198. Criteria for alert configurations include, without limitation, healthcare data of the patient 102 (e.g., a vital sign measured by a healthcare monitoring device 136), a lack of healthcare data of the patient 102 (e.g., a healthcare monitoring device 136 stops reporting measurements to the patient-side RPM application 120), a lack of responsiveness of the patient 102 (e.g., failure to acknowledge a reminder), and/or the like.

In some embodiments, when an alert is triggered by the patient-side RPM application 120, relevant medical information is presented to the patient 102. The relevant medical information is included in the alert configuration and/or determined by the patient-side RPM application based on the healthcare plan. The relevant medical information is presented visually and/or audibly to the patient 102. For example, the relevant medical information can include instructions for responding to emergencies, exigencies, and emergent situations that are detected by the patient-side RPM application 120 based on healthcare data of the patient 102.

In cases in which an alert is triggered while the patient device 110 is connected with the network 150, when the alert has a severity level of "critical," the patient-side RPM application 120 sends an immediate notification, such as a text message or automated phone call, to the healthcare provider 198. When the alert has a severity level of "high," the RPM application 120 presents an audio and/or video alert on the provider device 180. When the alert has a severity level of "low," the RPM application 120 causes the alert to be recorded in the datastore 172 so that the alert is presented to the healthcare provider 198 the next time the health care provider reviews the records of the patient 102.

In cases in which an alert is triggered while the patient device 110 is not connected with the provider-side RPM platform 168 (e.g., the network 150 does not provide connectivity to the provider-side RPM platform 168), the patient-side RPM application 120 logs all alerts that are triggered in the memory 118. When the patient device 110 is next connected with the provider-side RPM platform 168 via the network 150, the patient-side RPM application 120 notifies the provider-side RPM platform 168 of all alerts logged since the patient-side RPM application 120 last connected with the provider-side RPM platform 168.

The patient-side RPM application 120 analyzes the healthcare data received from the one or more healthcare monitoring devices 136 to detect whether an event defined by an event configuration in a healthcare plan has occurred. For example, an event configuration can be used to determine whether an emergent health condition of the patient 102 is occurring or has occurred, based on the healthcare data from one or more of the healthcare monitoring devices 136. If the event is detected, then the patient-side RPM application 120 can configure one or more healthcare monitoring devices 136 to measure vital signs of the patient 102 according to the event configuration. If the patient-side RPM application 120 determines that one healthcare monitoring device 136 can both detect the event and measure the vital sign(s) to be reported in response to detecting the event, then the patient-side RPM application 120 configures the healthcare monitoring device 136 to detect the event and to measure the vital sign(s) in response to detecting the event. For example, an event configuration can define an event as the pulse rate of the patient being less than a threshold and blood pressure of the patient is to be measured for three minutes in response to the pulse rate falling below the threshold. In such a case, the patient-side RPM application 120 configures a healthcare monitoring device 136 to monitor the pulse rate of the patient 102 and, in response to detecting that the pulse rate is less than the threshold, measure the blood pressure for three minutes and report the measurements to the patient-side RPM application 120. In another example, an event configuration can define an event as the patient climbing three stairs and the oxygenation level of the patient is to be measured for one minute in response to the patient climbing the three stairs. In such a case, the patient-side RPM application 120 configures a healthcare monitoring device 136 to monitor the movement of the patient 102 and, in response to detecting that the patient 102 climbs three stairs, measure the oxygenation level for one minute and report the measurements to the patient-side RPM application 120.

For example, a state configuration can define the state as the patient being asleep and the vital sign to be measured as the oxygenation level of the patient 102. If the patient-side RPM application 120 determines, based on data from a first healthcare monitoring device 136 that the patient 102 is in the sleep state, and, in response, the patient-side RPM application 120 configures the first healthcare monitoring device or a second healthcare monitoring device 136 to collect the oxygenation level of the patient 102 while the patient-side RPM application 120 continues to detect that the patient 102 is in the sleep state, based on data the patient-side RPM application 120 receives from the first healthcare monitoring device 136.

The patient-side RPM application 120 sends healthcare data received by the healthcare monitoring devices 136 to the provider-side RPM platform 168, when the patient device is connected with the network 150. The patient-side RPM application 120 sends the healthcare data based on the healthcare plan. In some examples, the healthcare plan indicates that some vital signs of patient 102 are to be checked and only reported when the vital signs are outside of a desired range, and the patient-side RPM application 120 compares the healthcare data to the desired range and determines whether to send the healthcare data to the provider-side RPM platform 168.

The patient-side RPM application 120 provides reminders to the patient 102 according to the healthcare plan assigned to the patient 102. In some examples, the reminders are presented to the patient 102 via the I/O device(s) 134 and can be presented concurrently (e.g., next to or overlaying) with a television program or other content being presented by the patient device 110 via the I/O device(s) 134.

The patient-side RPM application 120 can also receive and respond to a video call initiated by the provider-side RPM platform 168, when the patient-side RPM application 120 is connected with the provider-side RPM platform 168 via the network 150. The patient-side RPM application 120 can also initiate a video call to the provider-side RPM platform 168, when the patient-side RPM application 120 is connected with the provider-side RPM platform 168 via the network 150. The patient-side RPM application 120 outputs video images and sounds of the video call via the I/O device(s) 134. Similarly, the patient-side RPM application 120 accepts video images and sounds of the video call via the I/O device(s) 134. The patient-side RPM application 120 sends video and audio data via the network interface 116 and network 150 to the provider-side RPM platform 168. The patient-side RPM application 120 receives video and audio data for presentation to the patient 102 via the network interface 116 and network 150.

The I/O device(s) 134 collects images, video streams, and audio streams of the patient and supplies them to the patient-side RPM application 120. In some examples, the I/O device(s) includes a microphone. The I/O device(s) 134 can be activated when the patient 102 and the healthcare provider 198 are having a video call and used to supply video and audio of the patient for the video call. During the video call, I/O device(s) 134 can be used to capture images of the patient 102 and/or one or more of the healthcare monitoring devices 136 for reference by the healthcare provider 198.

The provider-side RPM platform 168 executes on the server 160. The provider-side RPM platform 168 enables the healthcare provider 198 to create, review, update, and/or delete a healthcare plan assigned to the patient 102. The provider-side RPM platform 168 presents one or more graphical user interfaces (GUIs), such as one or more create, review, update, and delete (CRUD) GUIs via which the healthcare provider 198 creates, reviews, updates, and/or deletes a healthcare plan assigned to the patient 102. The healthcare provider 198 can access the GUIs via the network 150 and the healthcare provider device 180. The provider-side RPM platform 168 retrieves the healthcare plan assigned to the patient 102, if any, from the datastore 172. In some examples, the provider-side RPM platform 168 also retrieves current and historic healthcare data of the patient 102 from the datastore 172 to present to the healthcare provider 198, so that the healthcare provider can refer to the healthcare data of the patient 102 when updating the healthcare plan assigned to the patient 102.

In some examples, the provider-side RPM platform 168 obtains one or more pre-configured healthcare plans, which are intended for persons having particular diseases or conditions, from a library of healthcare plans 174 and presents those one or more pre-configured healthcare plans to the healthcare provider 198. The healthcare provider 198 can then include or customize the one or more pre-configured healthcare plans to create the healthcare plan assigned to the patient 102. The provider-side RPM platform 168 selects the one or more pre-configured healthcare plans to obtain from the library of healthcare plans 174 based on input from the healthcare provider 198. When the healthcare provider 198 is satisfied with the new or updated healthcare plan to assign to the patient 102, the provider-side RPM platform 168 saves the new or updated healthcare plan assigned to the patient 102 to the datastore 172 and sends the healthcare plan to the patient-side RPM application 120 via the network 150. If the patient-side RPM application 120 is not connected with the provider-side RPM platform 168 via the network 150, the provider-side RPM platform 168 marks the updated healthcare plan as needing to be updated at the patient-side RPM application 120 when the patient-side RPM application 120 next connects with the provider-side RPM platform 168.

The provider-side RPM platform 168 receives a confirmation that the patient-side RPM application 120 received the new or updated healthcare plan from the patient-side RPM application 120. If the provider-side RPM platform 168 does not receive the confirmation from the patient-side RPM application 120, the provider-side RPM platform 168 can notify the healthcare provider 198 of that the patient-side RPM application 120 did not receive the new or updated healthcare plan. Optionally, the provider-side RPM platform 168 can send the healthcare plan assigned to the patient 102 to the patient-side RPM application 120 at a regular interval as a precaution against unauthorized changes at the patient device 110.

The provider-side RPM platform 168 receives healthcare data regarding the patient 102 from the patient-side RPM application 120. The provider-side RPM platform 168 receives the healthcare data via the network 150 and network interface 164. The provider-side RPM platform 168 saves the received healthcare data to the datastore 172.

The provider-side RPM platform 168 analyzes the healthcare data regarding the patient 102 and detects whether any alerts regarding the healthcare data regarding the patient 102 should be triggered. If the provider-side RPM platform 168 detects that an alert regarding the healthcare data regarding the patient 102 should be triggered, the provider-side RPM platform 168 sends the triggered alert to the healthcare provider 198 via a text message, email, automated phone call, or any technically feasible means.

The provider-side RPM platform 168 can initiate a video call to the patient 102, based on input from the healthcare provider 198. The provider-side RPM platform 168 can also receive a video call from the patient-side RPM application 120. The provider-side RPM platform 168 transmits a request to start a video call to the patient-side RPM application 120 via the network interface 164 and the network 150. The provider-side RPM platform 168 accepts video and audio data from the provider device 180.

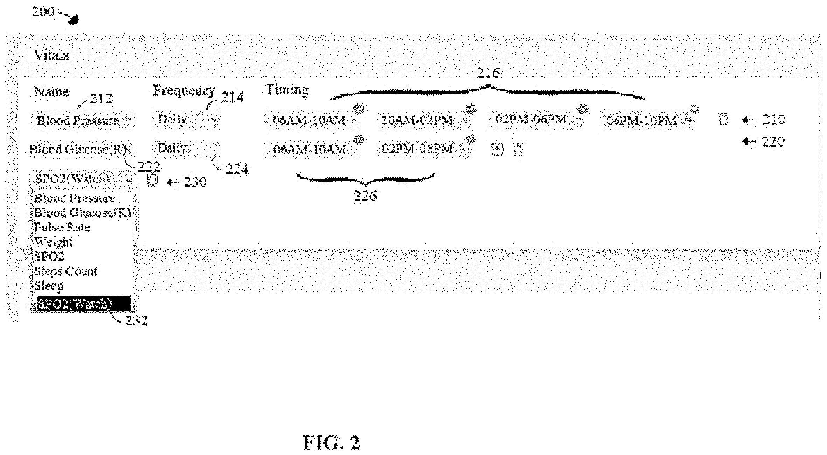

Figure 2 illustrates an example GUI 200 for configuring a healthcare plan, according to various embodiments of the present disclosure. The GUI 200 is presented by the provider-side RPM platform 168 to allow a healthcare provider 198 to specify one or more healthcare data monitoring configurations of a healthcare plan. Each row 210, 220, or 230 in the GUI 200 illustrates a vital sign of the patient 102 that is to be monitored according to the healthcare plan.

The entry at 212 shows that blood pressure is selected for monitoring in the displayed healthcare plan. The entry at 214 indicates the frequency at which the blood pressure is to be monitored. As shown via the entry at 214, the blood pressure is to be monitored daily. Although not shown, other monitoring intervals are possible, such as hourly, weekly, and/or the like. Additionally, the frequency field can indicate that the vital sign of that row is part of the criteria for an event configuration or a state configuration. The entries at 216 show that blood pressure is to be monitored between 6AM and 10PM. The entry at 222 shows that blood glucose(R) is selected for monitoring in the healthcare plan. The entry at 224 indicates that the blood glucose(R) is monitored daily, and the entries at 226 show that blood glucose(R) is to be monitored between 6AM and 10AM and between 2PM and 6PM. A drop-down list 232 is used by the healthcare provider 198 for selecting an additional vital sign to be monitored in the displayed healthcare plan. Once the healthcare provider 198 has selected the additional vital sign, the healthcare provider 198 selects a frequency and specific times for collecting the additional vital sign.

When a healthcare provider 198 selects that a vital sign is part of the criteria for an event configuration, additional fields are presented by the GUI 200 to enable the healthcare provider 198 to configure vital signs to be monitored or actions to be taken in response to detecting occurrence of the event or detecting healthcare data indicative of the event. When a healthcare provider 198 selects that a vital sign is part of the criteria for a state configuration, additional fields are presented by the GUI 200 to enable the healthcare provider 198 to configure vital signs to be monitored or actions to be taken in response to detecting the physiological state or detecting healthcare data indicative of the patient entering the physiological state.

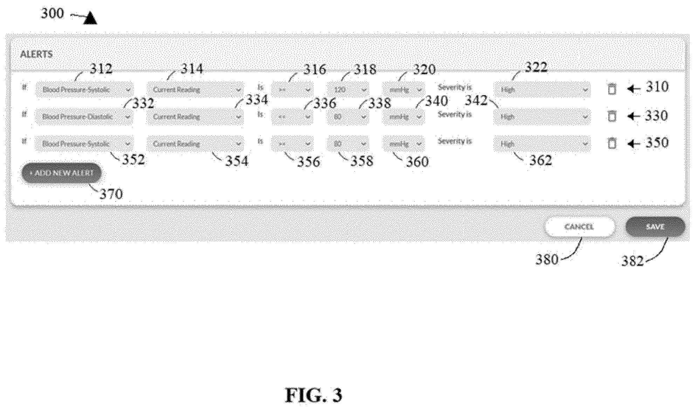

Figure 3 illustrates an example of a GUI 300 for configuring a healthcare plan, according to various embodiments of the present disclosure. The GUI 300 is presented by the provider-side RPM platform 168 via the provider device 180 to allow a healthcare provider 198 to specify one or more alert configurations of a healthcare plan. Each row 310, 330, 350 in the GUI 300 illustrates an alert configuration included in the displayed healthcare plan.

The entry at 312 shows systolic blood pressure is selected as the basis of the first alert of the displayed healthcare plan. The entry at 314 shows which reading or function of readings on which the first alert is based. As shown via the entry at 314, the current reading of the systolic blood pressure is the basis of the first alert. Although not shown, other readings or functions of readings are possible, such as maximum reading over last hour, average reading over last two hours, or difference from a previous reading. The entry at 316 shows the relationship of the vital sign to a standard, shown at 318 and 320, for the vital sign that triggers the first alert. As shown via the entry at 316, the first alert is triggered when the current systolic blood pressure is greater than or equal to the standard shown at 318 and 320. The entry at 318 shows the numerical value for the standard for the vital sign on which the first alert is based, and the entry at 320 shows the unit of measure for the standard. As shown via the entries at 318 and 320, the standard for the first alert is 120 mmHg for the current systolic blood pressure. The entry at 322 shows the relative severity (e.g., critical, high, or low) of the first alert. The severity level of the alert determines how an RPM application, such as the RPM application 120 responds to an alert. For example, an alert with a severity level of "critical" indicates that the RPM application 120 should provide an alert to the patient and contact emergency medical services; an alert with a severity level of "high" indicates that the RPM application 120 should provide an alert to a patient and call or page a healthcare provider; and an alert with a severity level of "low" indicates that the RPM application 120 should alert the patient and log the alert to be recorded with the healthcare data for the patient102. Each of the entries at 312, 314, 316, 318, 320, and 322 can be selected from a corresponding drop-down list.

The entry at 332 shows systolic blood pressure is selected as the basis of the second alert of the displayed healthcare plan. The entry at 334 shows which reading or function of readings on which the second alert is based. As shown via the entry at 334, the current reading of the systolic blood pressure is the basis of the second alert. The entry at 336 shows the relationship of the vital sign to a standard, shown at 338 and 340, for the vital sign that triggers the second alert. As shown via the entry at 336, the second alert is triggered when the current systolic blood pressure is less than or equal to the standard shown at 338 and 340. The entry at 338 shows the numerical value for the standard for the vital sign on which the second alert is based, and the entry at 340 shows the unit of measure for the standard. As shown via the entries at 338 and 340, the standard for the second alert is 80 mmHg for the current systolic blood pressure. The entry at 342 shows the relative severity (e.g., critical, high, or low) of the second alert. As shown via the entry at 322, the relative severity of the second alert of the displayed healthcare plan is high. Each of the entries at 332, 334, 336, 338, 340, and 342 can be selected from a corresponding drop-down list.

The entry at 352 shows systolic blood pressure is selected as the basis of the third alert of the displayed healthcare plan. The entry at 354 shows which reading or function of readings on which the third alert is based. As shown via the entry at 354, the current reading of the systolic blood pressure is the basis of the third alert. The entry at 356 shows the relationship of the vital sign to a standard, shown at 358 and 360, for the vital sign that triggers the third alert. As shown via the entry at 356, the third alert is triggered when the current systolic blood pressure is greater than or equal to the standard shown at 358 and 360. The entry at 358 shows the numerical value for the standard for the vital sign on which the third alert is based, and the entry at 360 shows the unit of measure for the standard. As shown via the entries at 358 and 360, the standard for the third alert is 80 mmHg for the current systolic blood pressure. The entry at 362 shows the relative severity (e.g., critical, high, or low) of the third alert. As shown via the entry at 322, the relative severity of the third alert of the displayed healthcare plan is high. Each of the entries at 352, 354, 356, 358, 360, and 362 can be selected from a corresponding drop-down list.

A button 370 enables the healthcare provider 198 to add a new alert to the displayed healthcare plan. Upon the healthcare provider 198 selecting the button 370, the provider-side RPM platform 168 adds a row to the GUI 300. The added row includes a set of drop-down lists for the healthcare provider 198 to use to select a vital sign, reading or function of readings, relationship to a standard, standard, unit of measure, and severity for the new alert. A cancel button 380 enables the healthcare provider 198 to exit the GUI without saving any changes to the alerts of the displayed healthcare plan. A save button 382 enables the healthcare provider 198 to save the changes to the displayed healthcare plan.

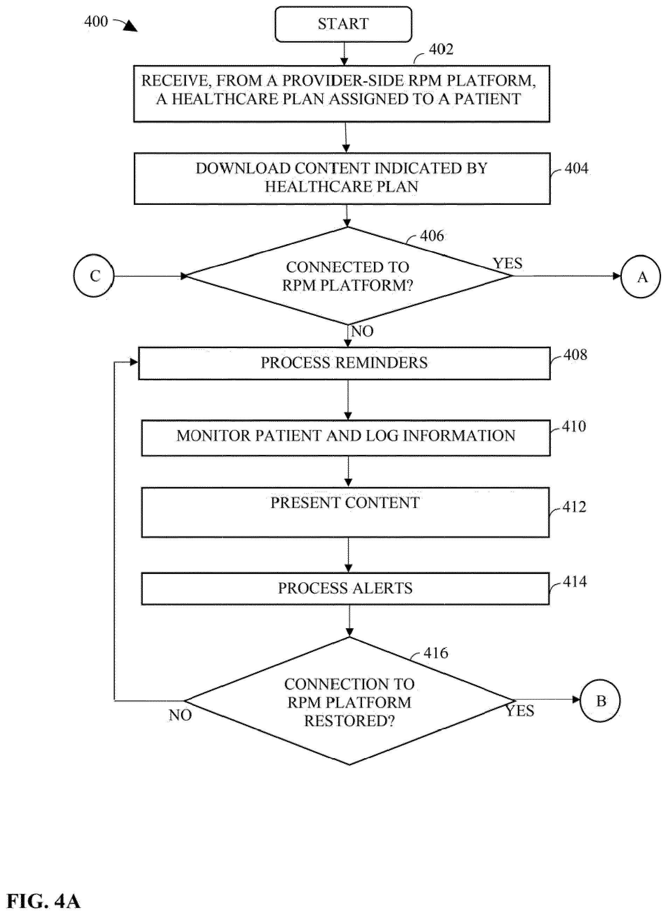

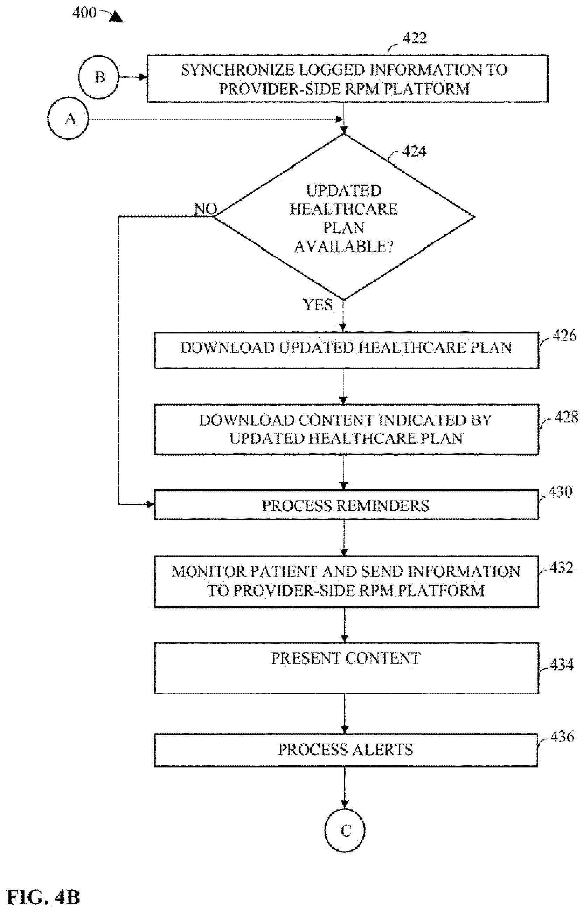

Figures 4A and 4B illustrate a flow diagram of method steps for providing remote patient assistance, according to various embodiments of the present disclosure. Although the method steps are described with respect to the systems of Figure 1, any system configured to perform the method steps, in any order, falls within the scope of the various embodiments. In some embodiments, method 400 is performed by the patient-side RPM application 120.

The method 400 begins at step 402, where a healthcare plan for a patient is received from a provider-side RPM platform. The health care plan includes one or more offerings of content, monitoring configurations, event configurations, state configurations, reminder configurations, and/or alert configurations, such as is described in greater detail above with respect to Figure 1. In some examples, the healthcare plan is received by an RPM application executing on a patient device, such as the patient-side RPM application 120 executing on patient device 110. In some examples, the RPM application acknowledges receipt of the health care plan. The RPM application parses the healthcare plan to determine whether the healthcare plan includes offerings of content; configurations for healthcare monitoring devices based on monitoring configurations, event configurations, and state configurations; reminder configurations, and/or alert configurations.

In step 404, content indicated by the healthcare plan is downloaded. The content can include videos, audio recordings, written instructions, questionnaires, relevant medical information, and the like. In some examples, the content is downloaded by the RPM application. In some examples, the content is identified by a link or URL, and the link or URL is used by an RPM application to retrieve and download the content.

In step 406, it is determined whether there is a connection to a provider-side RPM platform. In some examples, the RPM application determines whether the RPM application is connected to a provider-side RPM platform via a WAN using a network interface, such as the network interface 116. In some examples, checking whether there is a connection to a provider-side RPM platform includes determining whether a provider-side RPM platform, such as provider-side RPM platform 168, can be connected to by sending one or more test messages to the provider-side RPM platform and receiving acknowledgments of the test messages from the provider-side RPM platform. If there is no connection to a provider-side RPM platform, the method proceeds to step 408. If there is a connection to a provider-side RPM platform, the method proceeds to step 424, shown in Figure 4B.

In step 408, reminders are processed. The reminders are processed according to one or more reminder configurations included in the healthcare plan. For example, a reminder configuration can specify a reminder at a particular time of day, and the RPM application presents the reminder (e.g., shows a visible message and/or plays a sound on an I/O device) when that time of day occurs. Examples of reminders are a reminder to take medications or to perform some other activity at a particular time.

In step 410, the patient is monitored, and information is logged. In some examples, the RPM application monitors the patient, such as patient 102, using one or more healthcare monitoring devices, such as healthcare monitoring devices 136. In some examples, monitoring the patient includes configuring one or more healthcare monitoring devices, such as the healthcare monitoring devices 136, to collect healthcare data regarding the patient according to the healthcare plan. In some examples, the RPM application configures each of the one or more healthcare monitoring devices using one or more APIs provided by the healthcare monitoring device. In some examples, configuring a healthcare monitoring device includes configuring the healthcare monitoring device to detect an event or collect and report healthcare data indicative of an event per an event configuration in the healthcare plan. Examples of events include a vital sign of the patient going above or below a threshold, the patient falling, the patient beginning or ceasing activities (e.g., exercise, climbing stairs, eating, sleeping, walking, running), and/or the like. In some examples, configuring a healthcare monitoring device includes configuring the healthcare monitoring device to detect a state and/or collect and report healthcare data indicative of a physiological state per a state configuration in the healthcare plan. Examples of physiological states include being asleep, being in rapid eye movement (REM) during sleep, exercising, walking, climbing stairs, being in labor, and/or the like. In some examples, an RPM application executing on a patient device configures a healthcare monitoring device via a network interface. In some examples, configuring a healthcare monitoring device includes configuring the healthcare monitoring device per a monitoring configuration in the healthcare plan, such as to collect healthcare data for one or more vital signs at particular frequencies, collection times, and/or the like. Examples of healthcare data that can be collected include pulse rate, blood pressure, blood oxygenation, EKG, EEG, temperature, REM, physical activity (e.g., exercising, walking, climbing stairs), and/or the like. In some examples, the logged information includes healthcare data regarding the patient collected from one or more healthcare monitoring devices, such as the healthcare monitoring devices 136. In some examples, the logged information includes information collected from the patient, such as answers to questions on a questionnaire that is included in the healthcare plan.

In step 412, content is presented to the patient. In some examples, the RPM application selects content to present based on detecting an event, a state of the patient, an emergent condition, and/or the like. For example, in response to the RPM application determining that blood pressure of the patient is high (e.g., based on healthcare data from a healthcare monitoring device), a questionnaire can be presented to the patient including a question asking the patient if a medication has been taken. The content is presented by the RPM application using one or more suitable I/O devices 134. In some examples, offering the content includes displaying a user interface with a list of available and previously downloaded content from which the patient can select.

In step 414, one or more alerts are processed. In some examples, an RPM application analyzes the healthcare data received during step 410 to determine whether one or more alerts are to be triggered. The RPM application compares the healthcare data to the condition identified in each alert configuration included in the healthcare plan. When the healthcare data is consistent with the condition specified in a particular alert configuration, the RPM application generates an alert consistent with the severity level specified in the particular alert configuration. Depending on the severity level, the alert includes one or more of providing a notice to the patient, sending a notification to an RPM platform or a healthcare provider, dialing 911, and/or the like.

In step 416, it is determined whether the connection to the provider-side RPM platform is restored. In some examples, an RPM application executing on a patient device determines whether the connection to the provider-side RPM platform is restored using a network interface. In some examples, checking whether the connection to the provider-side RPM platform is restored includes determining whether a WAN connection is available. In some examples, checking whether there is a connection to a provider-side RPM platform includes determining whether a provider-side RPM platform can be connected to by sending one or more test messages to the provider-side RPM platform and receiving acknowledgments of the test messages from the provider-side RPM platform. If the connection to the provider-side RPM platform is not restored, the method proceeds to step 408. If the connection to the provider-side RPM platform is restored, the method proceeds to step 422, shown in Figure 4B.

Steps 408-414 are then repeated as needed to process reminders, monitor the patient and log additional healthcare information, present additional content to the patient, and/or process one or more additional alerts until connectivity to the provider-side RPM platform is restored. If the connection to the provider-side RPM platform is restored, the method proceeds to step 422, shown in Figure 4B.

Referring to Figure 4B, in step 422, logged information is synchronized to a provider-side RPM platform. In some examples, an RPM application, synchronizes logged healthcare information to a provider-side RPM platform, such as provider-side RPM platform 168, via a WAN. In some examples, synchronizing the logged healthcare information includes sending one or messages including the healthcare information logged during step 412 to the provider-side RPM platform and optionally receiving acknowledgments of the messages. Upon receiving an acknowledgment of a message, the RPM application can optionally delete the logged healthcare information corresponding to the acknowledged message and free memory used to store the logged healthcare information. If a message is not acknowledged, the RPM application sends an additional message(s) with the corresponding logged healthcare information at intervals until receiving an acknowledgment for at least one of the messages.

In step 424, it is determined whether an updated healthcare plan is available. In some examples, the RPM application determines whether an updated healthcare plan is available for the patient by sending a query to a provider-side RPM platform via a WAN. If an updated healthcare plan is available, the method proceeds to step 426. If an updated healthcare plan is not available, the method proceeds to step 430.

In step 426, the updated healthcare plan is downloaded. Step 426 is substantially the same as step 402.

In step 428, content indicated by the updated healthcare plan is downloaded. Step 428 is substantially the same as step 404.

In step 430, reminders are processed. Step 430 is substantially the same as step 408.

In step 432, the patient is monitored, and information is sent to the provider-side RPM platform. Similar to step 410, the RPM application monitors the patient using one or more healthcare monitoring devices. The RPM application then sends the healthcare information to the provider-side RPM platform via a WAN.

In step 434, content is presented to the patient. Step 434 is substantially the same as step 412.

In step 436, one or more alerts are processed. Step 436 is substantially the same as step 414. From step 436, the method proceeds to step 406, shown in Figure 4A.

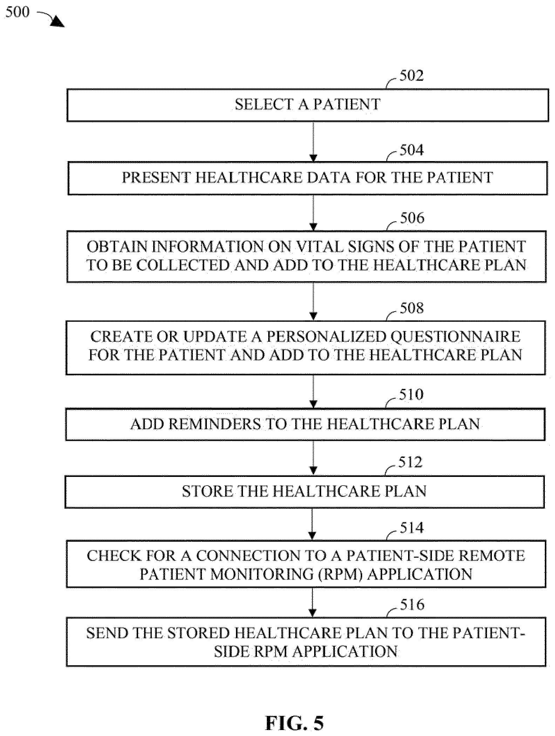

Figure 5 illustrates a flow diagram of method steps for managing a healthcare plan for providing remote patient assistance, according to various embodiments of the present disclosure. Although the method steps are described with respect to the systems and disclosures of Figures 1-3, any system configured to perform the method steps, in any order, falls within the scope of the various embodiments. In some embodiments, method 500 is performed by the provider-side RPM platform 168.

The method 500 begins at step 502, where a patient is selected. The patient, such as patient 102, is selected using an RPM platform, such as the provider-side RPM platform 168 executing on server 160. In some examples, a healthcare provider, such as healthcare provider 198, selects the patient by providing input to the RPM platform. For example, the healthcare provider can select the patient from a list of patients displayed by the RPM platform.

In step 504, healthcare data for the patient is presented. In some examples, the healthcare data is presented using an RPM platform via provider device, such as the provider device 180. In some examples, the healthcare data is retrieved from a datastore, such as the datastore 172. The healthcare data can represent records of medical conditions of the patient. The RPM platform receives the healthcare data from the RPM application and/or directly from one or more healthcare monitoring devices via a WAN. In some examples, the healthcare data is received in response to the RPM platform requesting the healthcare data and/or at periodic intervals.

In step 506, information on vital signs of a patient to be collected is obtained and added to a healthcare plan assigned to the patient. The healthcare plan includes one or more offerings of content, monitoring configurations, event configurations, state configurations, reminder configurations, and/or alert configurations, such as is described in greater detail above with respect to Figure 1. In some examples, a healthcare provider uses the RPM platform to create or update monitoring configurations of the healthcare plan assigned to the patient. In some embodiments, creating or updating the monitoring configurations of the healthcare plan includes adding or updating one or more vital signs to cause an RPM application, such as patient-side RPM application 120, to monitor the vital signs at particular frequencies, collection times, and/or the like. In some examples, the healthcare provider uses one or more GUIs, such as GUIs of Figures 2 and 3, presented by the RPM platform to create and update the monitoring configurations in the healthcare plan. In some examples, the healthcare provider creates or updates one or more alert configurations for inclusion in the healthcare plan.

In step 508, a personalized questionnaire for the patient is created or updated and added to the healthcare plan. In some examples, a healthcare provider uses the RPM platform to determine questions to include in the questionnaire and conditions in which the patient-side RPM application should present the questionnaire to the patient. In some examples, a healthcare provider uses the RPM platform to create multiple-choice questions (e.g., input questions and possible answers) in the questionnaire. In some examples, a healthcare provider uses the RPM platform to configure questions that depend on answers to previous questions, (e.g., questions about pain in particular locations that are asked when a patient has already answered a question saying they have pain). In some examples, a healthcare provider uses the RPM platform to select content to present to the patient in response to answers provided by the patient.

In step 510, reminders are added to the healthcare plan. In some examples, a healthcare provider uses the RPM platform to determine reminders (e.g., to take medication, connect a healthcare monitoring device to the patient-side RPM application so a vital sign can be monitored, or the like) for the patient and add those reminders to the healthcare plan. In some examples, a healthcare provider determines the reminders based on the healthcare data for the patient presented in step 504.

In step 512, the healthcare plan is stored. In some examples, the healthcare plan is stored in a datastore, such as datastore 172.

In step 514, a check for a connection to a patient-side RPM application is performed. In some examples, the provider-side RPM platform checks for a connection to the patient-side RPM application via a network interface, such as the network interface 164, and a network, such as the network 150. In some examples, the provider-side RPM platform checks for a connection to a patient-side RPM application that is associated with a patient to which the healthcare plan is assigned. In some examples, checking whether there is a connection to a patient-side RPM application includes determining whether the patient-side RPM application can be connected to by sending one or more test messages to the patient-side RPM application and receiving acknowledgments of the test messages from the patient-side RPM application. If there is not a connection to a patient-side RPM application, then step 514 is scheduled to be repeated at a later time. If there is a connection to a patient-side RPM application, then the method proceeds to step 516.

In step 516, the stored healthcare plan is sent to the patient-side RPM application. In some examples, the healthcare plan is sent to the patient-side RPM application via a network interface and a network. In some examples, the provider-side RPM platform receives a confirmation from the patient-side RPM application that the healthcare plan has been received. In some examples, the healthcare plan is sent to the patient-side RPM application in response to the healthcare provider completing creation or updating of the healthcare plan.

Steps 502-516 are then repeated as needed to select a patient and then create and/or update a healthcare plan for that patient.

In sum, a remote patient monitoring (RPM) application receives, from a provider-side RPM platform, a healthcare plan assigned to a patient. The patient-side RPM application downloads content identified in the healthcare plan. The patient-side RPM application operates in two modes depending upon whether the patient-side RPM application has network connectivity with the provider-side RPM platform. When the RPM application is not connected to the provider-side RPM platform, the RPM application processes reminders, monitors the patient, logs information on the patient, presents content, and processes alerts. When the connection is restored, the RPM application synchronizes logged information to the provider-side RPM platform and checks for an updated healthcare plan for the patient. And while the patient-side RPM application is connected to the provider-side RPM platform, the patient-side RPM application receives healthcare plans, downloads content indicated by the healthcare plans, processes reminders, monitors the patient, sends information on the patient to the provider-side RPM platform, presents content, and processes alerts.

At least one technical advantage of the disclosed techniques relative to the prior art is that, with the disclosed techniques, a patient without connectivity to a provider-side RPM platform can be monitored and receive remote patient assistance based on the monitoring and a healthcare plan personalized for the patient by a healthcare professional. This enables the patient to receive relevant and timely medical information even when the patient does not have access to a healthcare professional or other remote resources. Another technical advantage of the disclosed techniques relative to the prior art is that, with the disclosed techniques, a healthcare professional can update a healthcare plan for a patient without regard to whether the patient has connectivity (e.g., via a WAN) to the provider-side RPM platform to receive the updated healthcare plan. This enables the healthcare professional to update the healthcare plan at a time chosen by the healthcare professional while ensuring that the patient will receive the update as soon as the patient has connectivity to the provider-side RPM platform. These technical advantages provide one or more technological improvements over prior art approaches.
1. In various embodiments, a computer-implemented method for providing medical information, the method comprises receiving, from a provider-side side remote patient monitoring (RPM) platform, a healthcare plan assigned to a patient, and while disconnected from the provider-side RPM platform comprises monitoring the patient; in response to the monitoring, determining whether to provide the medical information to the patient; and presenting the medical information to the patient, wherein the medical information is based on the healthcare plan and the monitoring.
2. The method of clause 1, further comprising in response to receiving the healthcare plan, downloading the medical information identified in the healthcare plan.
3. The method of clause 1 or clause 2, wherein monitoring the patient comprises receiving an indication from the patient of an emergent health condition.
4. The method of any of clauses 1-3, wherein selection of the medical information is based on the indication from the patient of the emergent health condition.
5. The method of any of clauses 1-4, wherein the healthcare plan comprises a questionnaire, and the method further comprises: presenting one or more questions of the questionnaire to the patient; receiving one or more answers to the one or more questions from the patient; identifying additional medical information based on the one or more answers; and presenting the additional medical information to the patient.
6. The method of any of clauses 1-5, wherein monitoring the patient based on the healthcare plan comprises receiving data from one or more healthcare monitoring devices.
7. The method of any of clauses 1-6, wherein monitoring the patient based on the healthcare plan comprises logging healthcare data of the patient in memory while disconnected from the provider-side RPM platform.
8. The method of any of clauses 1-7, further comprising: in response to determining that a connection with the provider-side RPM platform is restored, synchronizing the logged healthcare data to the provider-side RPM platform.
9. The method of any of clauses 1-8, further comprising: in response to determining that a connection to the provider-side RPM platform is restored, receiving an updated healthcare plan assigned to the patient.
10. In various embodiments, one or more non-transitory computer-readable media storing instructions that, when executed by one or more processors, cause the one or more processors to perform the steps of: receiving, from a provider-side side remote patient monitoring (RPM) platform, a healthcare plan assigned to a patient; and while disconnected from the provider-side RPM platform: monitoring the patient; in response to the monitoring, determining whether to provide medical information to the patient; and presenting the medical information to the patient, wherein the medical information is based on the healthcare plan and the monitoring.
11. The one or more non-transitory computer-readable media of clause 10, wherein the steps further comprise in response to receiving the healthcare plan, downloading the medical information identified in the healthcare plan.
12. The one or more non-transitory computer-readable media of clause 10 or clause 11, wherein monitoring the patient comprises receiving an indication from the patient of an emergent health condition.
13. The one or more non-transitory computer-readable media of any of clauses 10-12, wherein selection of the medical information is based on the indication from the patient of the emergent health condition.
14. The one or more non-transitory computer-readable media of any of clauses 10-13, wherein the healthcare plan comprises a questionnaire and the steps further comprise: presenting one or more questions of the questionnaire to the patient; receiving one or more answers to the one or more questions from the patient; identifying additional medical information based on the one or more answers; and presenting the additional medical information to the patient.
15. The one or more non-transitory computer-readable media of any of clauses 10-14, wherein monitoring the patient based on the healthcare plan comprises receiving data from one or more healthcare monitoring devices.
16. The one or more non-transitory computer-readable media of any of clauses 10-15, wherein monitoring the patient based on the healthcare plan comprises logging healthcare data of the patient in memory while disconnected from the provider-side RPM platform.
17. The one or more non-transitory computer-readable media of any of clauses 10-16, wherein the steps further comprise: in response to determining that a connection with the provider-side RPM platform is restored, synchronizing the logged healthcare data to the provider-side RPM platform.
18. The one or more non-transitory computer-readable media of any of clauses 10-17, wherein the steps further comprise: in response to determining that a connection to the provider-side RPM platform is restored, receiving an updated healthcare plan assigned to the patient.
19. A patient-side device comprising: a memory storing instructions; and a processing system coupled to the memory that executes the instructions to perform steps comprising: receiving, from a provider-side side remote patient monitoring (RPM) platform, a healthcare plan assigned to a patient; and while disconnected from the provider-side RPM platform: monitoring the patient; in response to the monitoring, determining whether to provide medical information to the patient; and presenting the medical information to the patient, wherein the medical information is based on the healthcare plan and the monitoring.
20. The patient-side device of clause 19, wherein monitoring the patient comprises at least one of receiving an indication from the patient of an emergent health condition or receiving data from one or more healthcare monitoring devices.

Any and all combinations of any of the claim elements recited in any of the claims and/or any elements described in this application, in any fashion, fall within the contemplated scope of the present invention and protection.

The descriptions of the various embodiments have been presented for purposes of illustration but are not intended to be exhaustive or limited to the embodiments disclosed. Many modifications and variations will be apparent to those of ordinary skill in the art without departing from the scope and spirit of the described embodiments.

Aspects of the present embodiments may be embodied as a system, method, or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "module," a "system," or a "computer." In addition, any hardware and/or software technique, process, function, component, engine, module, or system described in the present disclosure may be implemented as a circuit or set of circuits. Furthermore, aspects of the present disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain or store a program for use by or in connection with an instruction execution system, apparatus, or device.

Aspects of the present disclosure are described above with reference to flowchart illustrations and/or block diagrams of methods, apparatus (systems) and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine. The instructions, when executed via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable gate arrays.

The flowchart and block diagrams in the figures illustrate the architecture, functionality, and operation of possible implementations of systems, methods, and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

While the preceding is directed to embodiments of the present disclosure, other and further embodiments of the disclosure may be devised without departing from the basic scope thereof, and the scope thereof is determined by the claims that follow.

## Claims

1. A computer-implemented method for providing medical information, the method comprising:
receiving, from a provider-side side remote patient monitoring, RPM, platform, a healthcare plan assigned to a patient; and
while disconnected from the provider-side RPM platform:
monitoring the patient;
in response to the monitoring, determining whether to provide the medical information to the patient; and
presenting the medical information to the patient, wherein the medical information is based on the healthcare plan and the monitoring.

2. The method of claim 1 further comprising:
in response to receiving the healthcare plan, downloading the medical information identified in the healthcare plan.

3. The method of claim 1 or 2, wherein monitoring the patient comprises receiving an indication from the patient of an emergent health condition.

4. The method of claim 3, wherein selection of the medical information is based on the indication from the patient of the emergent health condition.

5. The method of any one of claims 1-4, wherein the healthcare plan comprises a questionnaire, and the method further comprises:
presenting one or more questions of the questionnaire to the patient;
receiving one or more answers to the one or more questions from the patient;
identifying additional medical information based on the one or more answers; and
presenting the additional medical information to the patient.

6. The method of any one of claims 1-5, wherein monitoring the patient based on the healthcare plan comprises receiving data from one or more healthcare monitoring devices.

7. The method of any one of claims 1-6, wherein monitoring the patient based on the healthcare plan comprises logging healthcare data of the patient in memory while disconnected from the provider-side RPM platform.

8. The method of claim 7, further comprising:
in response to determining that a connection with the provider-side RPM platform is restored, synchronizing the logged healthcare data to the provider-side RPM platform.

9. The method of any one of claims 1-8, further comprising:
in response to determining that a connection to the provider-side RPM platform is restored, receiving an updated healthcare plan assigned to the patient.

10. One or more non-transitory computer-readable media storing instructions that, when executed by one or more processors, cause the one or more processors to perform the steps of:
receiving, from a provider-side side remote patient monitoring, RPM, platform, a healthcare plan assigned to a patient; and
while disconnected from the provider-side RPM platform:
monitoring the patient;
in response to the monitoring, determining whether to provide medical information to the patient; and
presenting the medical information to the patient, wherein the medical information is based on the healthcare plan and the monitoring.

11. The one or more non-transitory computer-readable media of claim 10, wherein monitoring the patient comprises receiving an indication from the patient of an emergent health condition, the steps further comprising selecting the medical information based on the indication from the patient of the emergent health condition.

12. The one or more non-transitory computer-readable media of claim 10 or 11, wherein the healthcare plan comprises a questionnaire and the steps further comprise:
presenting one or more questions of the questionnaire to the patient;
receiving one or more answers to the one or more questions from the patient;
identifying additional medical information based on the one or more answers; and
presenting the additional medical information to the patient.

13. The one or more non-transitory computer-readable media of any one of claims 10-12, wherein monitoring the patient based on the healthcare plan comprises:
receiving healthcare data from one or more healthcare monitoring devices;
logging the healthcare data in memory while disconnected from the provider-side RPM platform; and
in response to determining that a connection with the provider-side RPM platform is restored, synchronizing the logged healthcare data to the provider-side RPM platform.

14. A patient-side device comprising:
a memory storing instructions; and
a processing system coupled to the memory that executes the instructions to perform steps comprising:
receiving, from a provider-side side remote patient monitoring, RPM, platform, a healthcare plan assigned to a patient; and
while disconnected from the provider-side RPM platform:
monitoring the patient;
in response to the monitoring, determining whether to provide medical information to the patient; and
presenting the medical information to the patient, wherein the medical information is based on the healthcare plan and the monitoring.

15. The patient-side device of claim 14, wherein monitoring the patient comprises at least one of receiving an indication from the patient of an emergent health condition or receiving data from one or more healthcare monitoring devices.
